(19) Europäisches Patentamt
European Patent Office
Office européen des brevets

(11) Publication number: **0 010 361**
**B1**

(12) **EUROPEAN PATENT SPECIFICATION**

(45) Date of publication of patent specification: **27.04.83**

(21) Application number: **79301957.1**

(22) Date of filing: **20.09.79**

(51) Int. Cl.³: **C 07 D 233/84,**
C 07 D 403/04,
A 61 K 31/415

(54) **Bis-imidazoles, their preparation and pharmaceutical compositions containing them.**

(30) Priority: **22.09.78 US 944672**

(43) Date of publication of application:
**30.04.80 Bulletin 80/9**

(45) Publication of the grant of the patent:
**27.04.83 Bulletin 83/17**

(84) Designated Contracting States:
**BE CH DE FR GB IT LU NL SE**

(56) References cited:
**EP - A - 0 013 732**
**DE - A - 2 635 876**
**DE - C - 960 279**
**FR - A - 2 015 184**

**CHEMICAL ABSTRACTS, vol. 89, no. 15, 9th October 1978, page 586, no. 129450p and Formula Index, C16-z, July-December 1978, page 2198**
**Columbus, Ohio, U.S.A.**
**R. P. GUPTA et al.: "Heterocyclic systems containing a bridgehead nitrogen atom: part XXX. Reaction of 2-mercapto-4,5-dianisylimidazole and chloroacetic acid and alkyl halides"**

**The file contains technical information submitted after the application was filed and not included in this specification**

(73) Proprietor: **SMITHKLINE BECKMAN CORPORATION**
**1 Franklin Plaza**
**Philadelphia Pennsylvania 19101 (US)**

(72) Inventor: **Hill, David Taylor**
**109 Magnolia Place**
**North Wales Pennsylvania 19454 (US)**

(74) Representative: **Hargreaves, Gerald Henry, Dr. et al,**
**P.O.Box 39**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 010 361 B1

# Bis-imidazoles, their preparation and pharmaceutical compositions containing them.

This invention relates to use of certain bis(imidazoles) as anti-arthritic agents, to pharmaceutical compositions containing such compounds, to certain novel bis(imidazoles) and to a process for their preparation.

There are a number of ways of suppressing an arthritic response. One way is to reduce inflammation caused by local irritancy stimulated by the presence of foreign material, a second is to inhibit the deposition of immune complexes in, for example, joints, and a third is to regulate cell-mediated immunity.

We have now found that bis(imidazole) compounds of formula (I):—

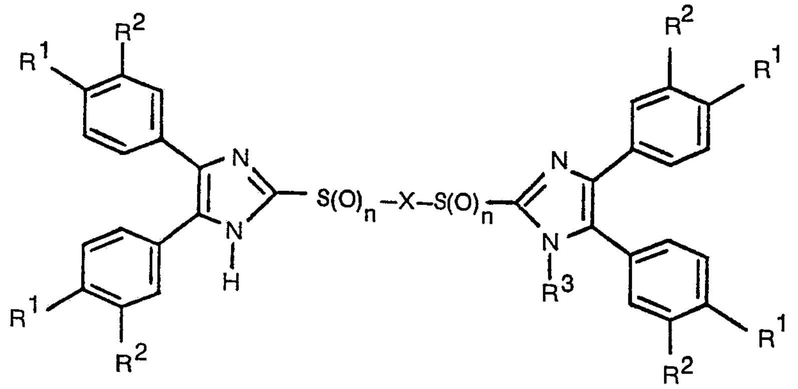

where $R^1$ is methoxy, methylthio, trifluoromethyl, chloro, fluoro or bromo; $R^2$ is hydrogen or with $R^1$ is methylenedioxy; $R^3$ is hydrogen or methyl; *n* is 0, 1 or 2; and X is $-(CH_2)_m-$ where *m* is 1 to 4, 1,2-propanediyl, 1,3-propan-2-oldiyl or 1,3-propan-2-onediyl and their pharmaceutically acceptable salts, provided that *n* is 0 when *m* is 1, relieve arthritis caused by local irritancy, the deposition of immune complexes and/or by cell-mediated immunity and are therefore of value in the treatment of this disease.

DE - A - 2 635 876 discloses certain anti-arthritic 4,5-diaryl substituted imidazoles which have a sulphur containing substituent at position 2. From the data in the document it can be seen that as the size of the substituent at position 2 increases so activity decreases. This observation teaches away from the compounds of formula (I) above in which the sulphur containing substituent at position 2 is large.

One compound of formula (I) viz. 2,2'[1,2-ethanediyl bis(thio)]bis[4,5-bis-(4-methoxyphenyl)-1H-imidazole] has been disclosed by Gupta *et al., Indian J. Chem* 16B: 329—331 (1978) but no biological activity has been ascribed to this compound and, in particular, it has never been suggested that it would be useful as an anti-arthritic agent.

Accordingly, in its broadest aspect, the invention provides a compound of formula (I) as previously defined or a pharmaceutically acceptable salt thereof for use as an anti-arthritic agent.

When X is 1,2-propanediyl, or 1,3 propan-2-oldiyl and $R^3$ is methyl, the compound contains an asymmetric carbon atom. It is to be understood that reference to such compounds in this specification includes reference to racemic mixtures and to optically pure enatiomers.

Preferred compounds for this use are those of formula (I) where $R^1$ is methoxy or fluoro, in particular when *n* is 0 and X is $-(CH_2)_m-$ (*m* being 1 or 2), 1,3-propan-2-oldiyl or 1,3-propan-2-onediyl.

Examples of compounds of formula (I) are:—

2,2'-[1,2-ethanediylbis(thio)]bis[4,5-bis-(4-fluorophenyl)-1H-imidazole],
2,2'-[1,2-ethanediylbis(thio)]bis[4,5-bis-(4-methoxyphenyl)-1H-imidazole],
2,2'-[methanediylbis(thio)]4,5-bis(4-fluorophenyl)-1H-imidazole],
2,2'-[1,2-ethanediylbis(sulfoxy)]bis[4,5-bis-(4-fluorophenyl)-1H-imidazole],
4,5-bis(4-fluorophenyl)-2-[2-(4,5-bis(4-fluorophenyl)-1H-2-imidazolylthio)ethylthio]-1-methylimidazole,
2,2'-[1,3-propan-2-oldiylbis(thio)]bis-[4,5-bis(4-fluorophenyl)-1H-imidazole], and
2,2'-[1,3-propan-2-onediylbis(thio)]bis[4,5-bis(4-fluorophenyl)-1H-imidazole].

Examples of pharmaceutically acceptable salts are maleate, fumarate, lactate, oxalate, methanesulfonate, ethanesulfonate, benzenesulfonate, tartrate, citrate, hydrochloride, hydrobromide, sulfate, phosphate and nitrate salts.

The ability of a compound to act as an anti-arthritic agent by regulating cell-mediated immunity is determined by the oxazolone-induced contact sensitivity test procedure in which changes in mouse paw oedema produced by administration of the test compound are measured. This test procedure is described by Griswold *et al., Cellular Immunology* 11: 198 (1974) and Griswold *et al., Inflammation*

2(4): 277 (1977). The implication of cell-mediated immune reactivity in rheumatoid arthritis is described by Basch *et al.*, J. *Rheumatology* 4:377 (1977).

Many of the compounds of formula (I) enhance the oxazolone-induced response at doses of from 12.5—100 mg/kg, orally. Specific examples of this activity exhibited by certain compounds of formula (I) appear in Table 1.

The anti-arthritic activity of salts of compounds of formula (I) is demonstrated by their ability to inhibit adjuvant induced polyarthritis in rats as measured by reduction of rat paw oedema at daily doses of about 12.5—100 mg/kg orally. This is an indication of the ability of the compound to reduce inflammation caused by local irritancy and to inhibit the deposition of immune complexes. In this test procedure, adjuvant arthritis is produced in rats by a single intradermal injection of 0.75 mg. of *Mycobacterium butyricum* suspended in white paraffin oil into left hindpaw footpad. The injection paw becomes inflamed (increased volume) and reaches maximum size within three to five days (primary lesion). The animals exhibit a decrease in body weight gain during this initial period. The adjuvant arthritis (secondary lesion) occurs after approximately ten days and is characterized by inflammation of the non-injected right hind leg, decrease in body weight and further increase in the volume of the injected left hind leg. Test compounds are administered daily beginning on the day of the adjuvant injection for seventeen days thereafter exclusive of days, 4, 5, 11 and 12. Anti-inflammatory activity is shown by a decrease in volume of the inflamed leg and anti-arthritic activity is shown by the ability of a compound under test to protect the animals against development of both primary and secondary lesions of adjuvant arthritis. Specific examples of this activity exhibited by certain salts of compounds of formula (I) appear in Table 2.

TABLE 1

OXAZOLONE INDUCED CONTACT SENSITIVITY

| COMPOUND OF EXAMPLE | Dose (mg/kg) (based on free base) | % Increase Over Controls of Mouse Paw Edema Volume |
|---|---|---|
| 8 (as the dihydrobromide) | 25 | 40 |
| 13 (as the dihydrochloride) | 25 | 62 |
| 14 (as the dihydrobromide) | 25 | 52 |
| 15 (as the dihydrochloride) | 25 | 72 |
| 18 (as the dihydrobromide) | 25 | 68 |

TABLE 2

ADJUVANT INDUCED ARTHRITIS

| COMPOUND OF THE EXAMPLE | Dose (mg/kg/day) | Injected (left) Hindleg Volume (cc.) | | Uninjected (right) Hindleg Volume (cc.) |
|---|---|---|---|---|
| | | Day 3 | Day ]6 | Day ]6 |
| 8 (as the dihydrobromide) | 50 | –24 | –34 | –43 |
| 13 (as the dihydrochloride) | 50 | –27 | –31 | NS* |
| 14 (as the dihydrobromide) | 50 | –19 | –16 | NS* |
| 15 (as the dihydrochloride) | 50 | –26 | –33 | –31 |
| 18 (as the dihydrobromide) | 50 | –34 | –45 | –30 |
| Prednisolone | 20 | –35 | –43 | –58 |

* NS= not significant

When using compounds of formula (I) and their pharmaceutically acceptable salts for medicinal purposes, they are preferably formulated in accordance with standard pharmaceutical practice as pharmaceutical compositions.

Accordingly, the invention further provides a pharmaceutical composition comprising a compound of formula (I) above or a pharmaceutically acceptable salt thereof, and a pharmaceutically acceptable carrier.

The compounds of formula (I) and their pharmaceutically acceptable salts are preferably administered systemically. Systemic administration can be achieved by rectal, oral or parenteral administration. A typical suppository formulation consists of the active compound with a binding and/or lubricating agent, for example gelatin, glyceryl monostearate, or glyceryl distearate, and low melting vegetable waxes or fats. Typical parenteral compositions consist of a solution or suspension of a compound of the invention in a sterile aqueous carrier or a parenterally acceptable oil, for example, peanut oil or olive oil.

Compounds of formula (I) and salts thereof which are active when administered orally may be presented in the form of syrups, tablets, capsules, troches and lozenges. A syrup formulation will generally consist of a suspension or solution of the compound in a liquid carrier, for example, ethyl alcohol, glycerine or water with a flavouring or colouring agent.

To obtain a stable water soluble dose form, a pharmaceutically acceptable salt, of a compound of formula (I), preferably a hydrochloride or sulfate, is dissolved in an aqueous solution of an organic or inorganic acid, for example a 0.3 M solution of succinic or, preferably, citric acid.

Preferably the compositions are in unit dose form, for example, a capsule or tablet, so that the patient can administer to himself a single dose.

Preferably each dosage unit contains from 25 mg. to 200 mg. of a compound of formula (I) or a salt thereof, expressed as the free base.

Where the composition is in the form of a capsule, a compound of formula (I) or a salt thereof in granular form optionally with a binding agent, is encapsulated in an edible shell, e.g. of gelatin.

Where the composition is in the form of a tablet, any suitable pharmaceutical carrier routinely used for preparing solid formulations can be used. Examples of such carriers include, magnesium stearate, stearic acid, terra alba, agar, pectin, acacia, lactose, sucrose and talc. The amount of solid carrier can be varied widely, but preferably will be from 25 mg. to 1 g.

It will be recognised that the amount of a compound of formula (I) or a salt thereof administered to a patient will depend upon the severity of the condition being treated as well as the age and weight of the patient and the activity of the compound or salt selected for use. A preferred daily dosage regimen for an adult human is from 75—600 mg.

With the exception of 2,2′[1,2-ethanediylbis(thio)] bis [4,5-bis(4-methoxyphenyl)-1H-imidazole] compounds of formula (I) are novel.

Accordingly the invention provides a compound of formula (I)

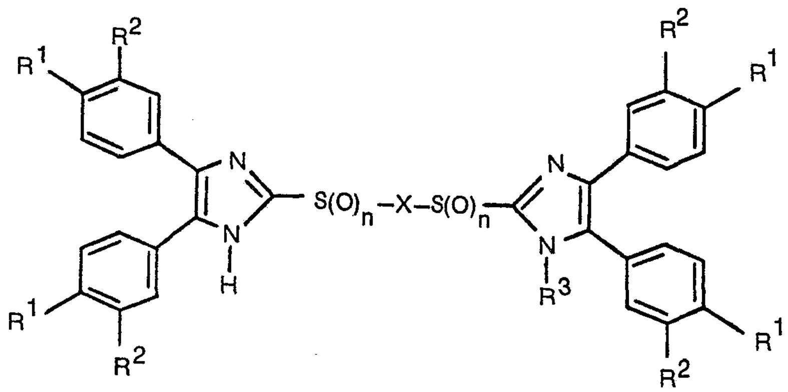

where $R^1$ is methoxy, methylthio, trifluoromethyl, chloro, fluoro or bromo; $R^2$ is hydrogen or with $R^1$ is methylenedioxy; $R^3$ is hydrogen or methyl; *n* is 0, 1 or 2; and X is $-(CH_2)_m-$ where *m* is 1 to 4, 1,2-propanediyl, 1,3-propan-2-oldiyl or 1,3-propan-2-onediyl; and its pharmaceutically acceptable salt provided that *n* is 0 when *m* is 1 and when $R^1$ is methoxy and *n* is 0 *m* is not 2.

Preferences with respect to $R^1$ and X are as previously disclosed.

The compounds of the invention can be prepared by a process which comprises:—

a) reacting a compound of formula (II):—

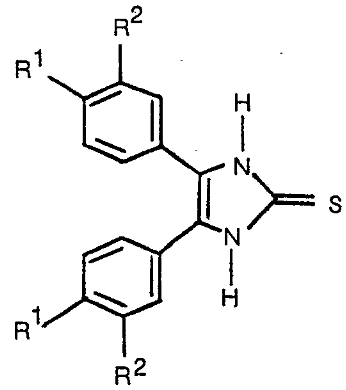

where $R^1$ and $R^2$ are as defined with reference to formula (I) with a reagent which introduces the group X;

b) optionally reacting the product of step a) with a methyl halide;

c) optionally oxidizing the product of step a) or step b) to a sulfoxide or sulfone.

Examples of reagents which introduce the group X are an appropriate $\alpha,\omega$-dihaloalkane, a 1,3-dihalopropan-2-ol, or a 1,3-dihalopropan-2-one, that is to say a compound of formula (III):—

YXY
(III)

where X is as defined with reference to formula (I) and Y is halogen preferably chlorine or bromine. The reaction is preferably carried out in a polar organic solvent, for example methanol, ethanol, dioxane or N,N-dimethylformamide, at a temperature of from about ambient temperature to the boiling point of the solvent.

When the reagent which introduces the group X is a compound of formula (III) the corresponding dihydrohalide salt will in general be produced. This salt can be isolated by standard techniques, and can be converted to the free base by known methods, for example by reaction with a base.

The compounds of formula (I) where $R^3$ is methyl can be prepared by reacting the corresponding compounds of formula (I) where $R^3$ is hydrogen with a base, for example sodium hydride, and methyl iodide.

The compounds of formula (I) (sulfoxide compounds) where *n* is 1, can be prepared by oxidizing the corresponding compound of formula (I), where *n* is 0 according to known methods with, for example, m-chloroperbenzoic acid.

The compounds of formula (I) where *n* is 2 (sulfone compounds) can be prepared by oxidizing the corresponding compounds of formula (I), where *n* is 0 according to known methods, with for example, hydrogen peroxide.

The thione starting materials of formula (II) can be prepared from the appropriately substituted benzoins, which are known or can be prepared by known methods, by reaction of the benzoin with thiourea in N,N-dimethylformamide, hexanol or a similar solvent.

The pharmaceutically acceptable salts of the compounds of the formula (I) can be formed with organic or inorganic acids by known methods. For example, the base can be reacted with an inorganic or organic acid in a water-miscible solvent, for example ethanol, and the salt can then be isolated by evaporating the solvent, or in a water-immiscible solvent in which the acid is soluble, for example, diethylether or chloroform, from which the salt precipitates or from which the salt can be isolated by evaporating the solvent.

The following Examples illustrates the invention. All temperatures are in degrees Centrigrade (°C).

### Example 1

2,2′-[1,2-Ethanediylbis(thio)]bis[4,5-bis(4-bromophenyl)-1H-imidazole]

A mixture of 4,4′-dibromobenzoin (25 g, 0.068 mol) and thiourea (10.3 g, 0.14 mol) in hexanol (200 ml) was refluxed with azeotropic distillation of water for three hours. The solution was then cooled and ethanol (300 ml) was added. The mixture was further cooled and the small needles which formed were collected and washed with cold ethanol then ether and dried. Recrystallization of the product from ethyl acetate gave 4,5-bis-(4-bromophenyl)-1H-imidazole-2-thione, m.p. 288°.

A mixture of 4,5-bis(4-bromophenyl)-1H-imidazole-2-thione (10 g, 0.024 mol) and 1,2-dibromoethane (2.3 g. 0.012 mol) in ethanol (100 ml) was heated at reflux temperature for eleven hours and then cooled to ambient temperature. The resulting product was removed by filtration, washed with ether and recrystallized from methanol to give the title compound as its dihydrobromide salt, m.p. 244—251°.

$C_{32}H_{22}Br_4N_4S_2 \cdot 2\ HBr$

| | | | |
|---|---|---|---|
| Calculated: | 38.13% C; | 2.40% H; | 5.56% N |
| Found: | 37.94% C; | 2.53% H; | 5.52% N |

2,2'-[1,2-Ethanediylbis(thio)]bis[4,5-bis(4-bromophenyl)-1H-imidazole] dihydrobromide (ca. 65 g) was slurried with warm ethanol (150 ml) and 5% aqueous sodium carbonate (500 ml) was added. The mixture was filtered and the precipitate was washed with water then ethanol, air-dried and recrystallized to give the title compound.

Example 2

2,2'-[1,2-Ethanediylbis(thio)]bis[4,5-bis(4-trifluoromethylphenyl)-1H-imidazole]

A solution of p-trifluoromethylbenzaldehyde (50 g, 0.24 mol), 3-ethyl-5-(2-hydroxyethyl)-4-methyl-1,3-thiazolium bromide (3.6 g, 0.014 mol) and triethylamine (8.8 g, 0.087 mol) in ethanol (200 ml) was refluxed 1.5 hours under an argon atmosphere. After cooling, the mixture was poured into water and extracted immediately into chloroform, taking precautions to exclude air as much as possible. The chloroform extracts were washed with 5% aqueous sodium bicarbonate solution and water and then dried ($MgSO_4$). Removal of the solvent at reduced pressure gave a nearly quantitative yield of crude 4,4'-di(trifluoromethyl)benzoin which was used without further purification.

A mixture of 4,4'-di(trifluoromethyl)benzoin (50 g, 0.15 mol) and thiourea (15.2 g, 0.2 mol) in dimethylformamide (350 ml) was refluxed under argon for three hours. The cooled solution was poured into 1 liter of water and the resulting solid was collected, washed with water, air-dried and recrystallized from ethanol to give 4,5-bis-(4-trifluoromethylphenyl)-1H-imidazole-2-thione, m.p. 312—315°.

A mixture of 4,5-bis(4-trifluoromethylphenyl)-1H-imidazole-2-thione (8 g, 0.02 mol) and 1,2-dibromoethane (2.2 g, 0.017 mol) in ethanol (100 ml) was refluxed overnight. The mixture was cooled and the precipitate was removed by filtration and recrystallized from ethanol to give the title compound as its dihydrobromide salt, m.p. 252—254°.

$C_{36}H_{22}F_{12}N_4S_2 \cdot 2\ HBr$

| | | | | |
|---|---|---|---|---|
| Calculated: | 44.83% C; | 2.51% H; | 5.81% N; | 16.57% Br |
| Found: | 44.85% C; | 2.68% H; | 5.68% N; | 16.37% Br |

2,2'-[1,2-Ethanediylbis(thio)]bis[4,5-bis(4-trifluoromethylphenyl)-1H-imidazole] dihydrobromide was converted to the title compound by the procedure described in Example 1.

Example 3

2,2'-[1,2-Ethanediylbis(thio)]bis[4,5-bis(4-methoxyphenyl)-1H-imidazole]

4,5-Bis(4-methoxyphenyl)-1H-imidazole-2-thione, m.p. 265°, was prepared by substitution of an equivalent amount of 4,4'-dimethoxybenzoin in either of the procedures for preparing imidazole thiones described in Examples 1 and 2.

A mixture of 4,5-bis(4-methoxyphenyl)-1H-imidazole-2-thione (31.2 g, 0.1 mol) and 1,2-dibromoethane 9.3 g, 0.05 mol) in ethanol (300 ml) was refluxed for four hours. After cooling, the solvent was removed at reduced pressure and the resulting solid was treated with ether and then filtered. The solid product was recrystallized several times from ethanol to give the title compound as its dihydrobromide salt (hydrate), m.p. 228°.

$C_{36}H_{34}N_4O_4S_2 \cdot 2\ HBr \cdot 1.5\ H_2O$

| | | | |
|---|---|---|---|
| Calculated: | 51.44% C; | 4.68% H; | 6.67% N |
| Found: | 51.37% C; | 4.81% H; | 6.52% N |

2,2'-[1,2-Ethanediylbis(thio)]bis[4,5-bis(4-methoxyphenyl)-1H-imidazole]dihydrobromide is converted to the title compound by the procedure described in Example 1.

Example 4

2,2'-[1,2-Ethanediylbis(thio)]bis[4,5-bis(4-chlorophenyl)-1H-imidazole]

4,5-Bis(4-chlorophenyl)-1H-imidazole-2-thione was prepared by substitution of an equivalent amount of 4,4'-dichlorobenzoin in either of the procedures for preparing imidazole thiones described in Examples 1 and 2.

A mixture of 4,5-bis(4-chlorophenyl)-1H-imidazole-2-thione (20 g, 0.062 mol) and 1,2-dibromoethane (5.8 g, 0.031 mol) in ethanol (200 ml) was refluxed for 6.5 hours. The resulting solid product was removed by filtration and washed with ethanol and then ether. Recrystallization from methanol gave the title compound as its dihydrobromide salt (hydrate), m.p. 251—252°.

$C_{32}H_{22}Cl_4N_4S_2 \cdot 2\ HBr \cdot 2.5\ H_2O$

| | | | | |
|---|---|---|---|---|
| Calculated: | 43.91% C; | 3.11% H; | 6.40% N; | 7.40% S |
| Found: | 43.81% C; | 3.25% H; | 6.36% N; | 7.54% S |

2,2′-[1,2-Ethanediylbis(thio)]bis[4,5-bis(4-chlorophenyl)-1H-imidazole]dihydrobromide is converted to the title compound by the procedure described in Example 1.

Example 5

2,2′-[1,2-Ethanediylbis(thio)]bis[4,5-bis(4-fluorophenyl)-1H-imidazole]

4,5-Bis(4-fluorophenyl)-1H-imidazole-2-thione, m.p. 309—311°, was prepared by substitution of an equivalent amount of 4,4′-difluorobenzoin in either of the procedures for preparing imidazole thiones described in Examples 1 and 2.

A mixture of 4,5-bis(4-fluorophenyl)-1H-imidazole-2-thione (15.2 g, 0.053 mol) and 1,2-dibromoethane (4.9 g, 0.026 mol) in dioxane (300 ml) was refluxed for 2.5 hours and then cooled. The resulting solid was removed by filtration, then washed with ether and recrystallized from ethanol-ether-dimethylformamide (35:60:5) to give the title compound as its dihydrobromide salt (hydrate), m.p. 302—304°.

$C_{32}H_{22}F_4N_4S_2 \cdot 2\ HBr \cdot 2\ H_2O$

| | | | | |
|---|---|---|---|---|
| Calculated: | 48.01% C; | 3.59% H; | 7.00% N; | 19.96% Br |
| Found: | 48.24% C; | 3.83% H; | 6.77% N; | 19.57% Br |

2,2′-[1,2-Ethanediylbis(thio)]bis[4,5-bis(4-fluorophenyl)-1H-imidazole]dihydrobromide was converted to the title compound by the procedure described in Example 1, m.p. 307—310°.

$C_{32}H_{22}F_4N_4S_2$

| | | | |
|---|---|---|---|
| Calculated: | 63.77% C; | 3.68% H; | 9.30% N |
| Found: | 63.37% C; | 3.84% H; | 9.12% N |

A solution 2,2′-[1,2-ethanediylbis(thio)]bis-[4,5-bis(4-fluorophenyl-1H-imidazole]dihydrobromide (10 g) in methanol (400 ml) was passed through a column of AGl—X10 analytical grade anion exchange resin, chloride form (200 g) to give, after removal of the solvent and recrystallization from methanol, the title compound as its dihydrochloride salt (hydrate), m.p. 190—195° (dec.).

$C_{32}H_{22}F_4N_4S_2 \cdot 2\ HCl \cdot H_2O$

| | | | |
|---|---|---|---|
| Calculated: | 55.41% C; | 3.78% H; | 8.07% N |
| Found: | 54.95% C; | 4.02% H; | 8.05% N |

A mixture of 4,5-bis(4-fluorophenyl)-1H-imidazole-2-thione (10 g, 0.034 mol), 1,2-dichloroethane (1.7 g, 0.017 mol) in N,N-dimethylformamide (45 ml) was stirred for 24 hours at 80° and then cooled. The precipitate was harvested and recrystallized from methanol to give the trihydrate, m.p. 234—236°.

$C_{32}H_{22}F_4N_4S_2 \cdot 2\ HCl \cdot 3\ H_2O$

| | | | |
|---|---|---|---|
| Calculated: | 52.68% C; | 4.14% H; | 7.68% N |
| Found: | 52.95% C; | 3.79% H; | 7.82% N |

Example 6

2,2′[1,3-Propanediylbis(thio)]bis[4,5-bis(4-fluorophenyl)-1H-imidazole]

A mixture of 4,5-bis(4-fluorophenyl)-1H-imidazole-2-thione (8 g, 0.028 mol) and 1,3-dibromopropane (2.8 g, 0.014 mol) in dioxane (175 ml) was refluxed for 2.5 hours. After cooling, ether (300 ml)

was added and the resulting white solid was collected by filtration, washed with ether and recrystallized from dioxane-ether to give the title compound as its dihydrobromide salt (hydrate), m.p. 176—178°.

$C_{33}H_{24}F_4N_4S_2 \cdot 2\ HBr \cdot 1.5\ H_2O$

| | | | | |
|---|---|---|---|---|
| Calculated: | 49.20% C; | 3.63% H; | 6.96% N; | 19.84% Br |
| Found: | 49.38% C; | 3.88% H; | 6.70% N; | 20.08% Br |

2,2'-[1,3-Propanediylbis(thio)]bis[4,5-bis(4-fluorophenyl)-1H-imidazole]dihydrobromide is converted to the title compound by the procedure described in Example 1.

## Example 7

2,2'-[1,4-Butanediylbis(thio)]bis[4,5-bis(4-fluorophenyl)-1H-imidazole]

A mixture of 4,5-bis(4-fluorophenyl)-1H-imidazole-2-thione (5.8 g, 0.02 mol) and 1,4-dibromobutane (4.3 g, 0.02 mol) in dioxane (75 ml) was refluxed one hour. After cooling, ether was added and the solid which formed was removed by filtration. This product was air dried and recrystallized from isopropanol to give the title compound as its dihydrobromide salt (hydrate), m.p. 172—174°.

$C_{34}H_{26}F_4N_2S_2 \cdot 2\ HBr \cdot H_2O$

| | | | |
|---|---|---|---|
| Calculated: | 50.38% C; | 3.73% H; | 6.91% N; |
| Found: | 50.30% C; | 3.68% H; | 6.88% N; |

2,2'-[1,4-Butanediylbis(thio)]bis[4,5-bis(4-fluorophenyl)-1H-imidazole dihydrobromide is converted to the title compound by the procedure described in Example 1.

## Example 8

2,2'-[Methanediylbis(thio)]bis[4,5-bis(4-fluorophenyl)-1H-imidazole]

A mixture of 4,5-bis(4-fluorophenyl)-1H-imidazole-2-thione (8 g, 0.028 mol) and dibromomethane (2.4 g, 0.014 mol) in ethanol (40 ml) was refluxed overnight. The mixture was cooled and the precipitate was removed by filtration. Recrystallization of the solid product from ethanol gave the title compound as its dihydrobromide salt, m.p. 265°.

$C_{36}H_{20}F_4N_4S_2 \cdot 2\ HBr$

| | | | |
|---|---|---|---|
| Calculated: | 49.61% C; | 2.95% H; | 7.47% N |
| Found: | 44.20% C; | 3.34% H; | 7.68% N |

2,2'-[1,2-Methanediylbis(thio)]bis[4,5-bis(4-fluorophenyl)-1H-imidazole dihydrobromide is converted to the title compound by the procedure described in Example 1.

## Example 9

2,2'-[1,2-Ethanediylbis(thio)]bis[4,5-bis(3,4-methylenedioxyphenyl)-1H-imidazole]

4,5-Bis)3,4-methylenedioxyphenyl)-1H-imidazole-2-thione was prepared by substitution of an equivalent amount of 4,4'-di(3,4-methylenedioxy)benzoin in either of the procedures for preparing imidazone thiones described in Examples 1 and 2.

A mixture of 4,5-bis(3,4-methylenedioxyphenyl)-1H-imidazole-2-thione (21.5 g, 0.063 mol) and 1,2-dibromoethane (5.9 g, 0.03 mol) in dioxane (400 ml) was refluxed for four hours and then cooled. The precipitate was removed by filtration and recrystallized from methanol to give the title compound as its dihydrobromide salt (hydrate), m.p. 188—190°.

$C_{36}H_{26}N_4O_8S_2 \cdot 2\ HBr \cdot 2.5\ H_2O$

| | | | | |
|---|---|---|---|---|
| Calculated: | 47.32% C; | 3.64% H; | 6.13% N; | 17.49% Br |
| Found: | 47.10% C; | 3.58% H; | 6.00% N; | 17.82% Br |

2,2'-[1,2-Ethanediylbis(thio)]bis[4,5-bis(3,4-methylenedioxy)-1H-imidazole]dihydrobromide is converted to the title compound by the procedure described in Example 1.

Example 10

2,2'[1,3-Propanediylbis(thio)]bis[4,5-bis(4-methoxyphenyl)-1H-imidazole]

A mixture of 4,5-bis(4-methoxyphenyl)-1H-imidazole-2-thione (15.6 g, 0.05 mol) and 1,3-dibromopropane (5 g, 0.025 mol) in ethanol (150 ml) was refluxed for three hours. After cooling, the resulting solid was removed by filtration and discarded. The solvent was removed at reduced pressure to give a yellow foam which was then treated with chloroform-ether. The resulting solid material was removed by filtration, dissolved in chloroform and washed with 10% aqueous sodium hydroxide solution. The solvent was removed at reduced pressure to give the title compound.

The title compound was dissolved in ethanol and 48% hydrobromic acid was added. Ether was added and the salt was collected and recrystallized from ethanol-ether to give the title compound as its dihydrobromide salt (hydrate) m.p. 128—130°.

$C_{37}H_{36}N_4O_4S_2 \cdot 2\ HBr \cdot 5\ H_2O$

| | | | | |
|---|---|---|---|---|
| Calculated: | 48.48% C; | 5.27% H; | 6.11% N; | 17.44% Br |
| Found: | 48.43% C; | 5.15% H; | 6.05% N; | 18.16% Br |

Example 11

2,2'-[1,2-Ethanediylbis(thio)]bis[4,5-bis(4-methylthiophenyl)-1H-imidazole]

4,5-Bis-(4-methylthiophenyl)-1H-imidazole-2-thione, m.p. 296°, was prepared by substitution of an equivalent amount of 4,4'-dimethylthiobenzoin in either of the procedures for preparing imidazole thiones described in Examples 1 and 2.

A mixture of 4,5-bis(4-methylthiophenyl)-1H-imidazole-2-thione (16.2 g, 0.04 mol) and 1,2-dibromoethane (8.8 g, 0.04 mol) in ethanol (250 ml) was refluxed four hours. After cooling, the precipitate was removed by filtration and recrystallized from ethanol to give the title compound, m.p. 237—238°.

$C_{36}H_{34}N_4S_6$

| | | | |
|---|---|---|---|
| Calculated: | 60.47% C; | 4.79% H; | 7.84% N |
| Found: | 60.74% C; | 5.03% H; | 7.77% N |

Example 12

2,2'-[1,2-Ethanediylbis(sulfonyl)]bis[4,5-bis(4-fluorophenyl-1H-imidazole]

(A) A mixture of 2,2'-[1,2-ethanediylbis(thio)]-bis[4,5-bis(4-fluorophenyl)-1H-imidazole] (3.0 g, 0.005 mol) and 30% hydrogen peroxide (2.8 g, 0.025 mol) in glacial acetic acid (100 ml) was heated at 70° for three hours and then cooled to ambient temperature. The resulting precipitate was filtered, washed with hexane and recrystallized from acetonitrile to give the title compound, m.p. 250—252°.

$C_{32}H_{22}F_4N_4O_4S_2$

| | | | |
|---|---|---|---|
| Calculated: | 57.48% C; | 3.62% H; | 8.38% N |
| Found: | 57.70% C; | 3.41% H; | 8.48% N |

(B) A mixture of 2,2'-[1,2-ethanediylbis(thio)]-bis[4,5-bis(4-fluorophenyl-1H-imidazole] (13.4 g, 0.022 mol) and 30% hydrogen peroxide (12.6 g, 0.112 mol) in glacial acetic acid was stirred above 0° for four hours and then cooled. The precipitate was removed by filtration, washed with hexane and dried *in vacuo* to give the title compound, m.p. 246—248°.

Example 13

2,2'-[1-2-Ethanediylbis(sulfoxy)]bis[4,5-bis(4-fluorophenyl)-1H-imidazole]

m-Chloroperbenzoic acid (3.5 g, 0.017 mol) was added to a solution of 2,2'-[1,2-ethanediylbis(thio)]bis-[4,5-bis(4-fluorophenyl)-1H-imidazole] (5.0 g, 0.008 mol) in glacial acetic acid (500 ml) and the mixture was stirred overnight at ambient temperature. The mixture was filtered and the residue was washed with water, dried *in vacuo* and recrystallized from dimethylformamide-ethanol to give the title compound, m.p. 175—180° (dec.).

$C_{32}H_{22}F_4N_4O_2S_2$

| | | | |
|---|---|---|---|
| Calculated: | 60.56% C; | 3.49% H; | 8.83% N |
| Found: | 60.40% C; | 3.62% H; | 8.96% N |

The title compound was converted to the corresponding dihydrochloride salt according to the procedure described in Example 5, m.p. 273—275°.

$C_{32}H_{22}F_4N_4O_2S_2 \cdot 2\ HCl$

| | | | |
|---|---|---|---|
| Calculated: | 54.32% C; | 3.42% H; | 7.92% N |
| Found: | 54.62% C; | 3.32% H; | 8.08% N |

Example 14

4,5-Bis(4-fluorophenyl-2-[2-(4,5-bis(4-fluorophenyl)-1H-2-imidazolylthio)ethylthio]-1-methylimidazole

A suspension of 2,2′-[1,2-ethanediylbis(thio)]bis-[4,5-bis(4-fluorophenyl)-1H-imidazole (6.0 g, 0.01 mol) in N,N-dimethylformamide (50 ml) was treated with sodium hydride (0.48 g, 0.01 mol) under an argon atmosphere. Methyl iodide (1.4 g, 0.01 mol) in N,N-dimethylformamide (10 ml) was added and the mixture was allowed to stir for one hour. The mixture was cooled, filtered and the filtrate was extracted with hexane and poured into water (125 ml). The precipitate was collected, washed with water, dried and chromatographed on a silica gel column with chloroform/ether (gradient) as eluant to give the title compound.

4,5 - Bis - (4-fluorophenyl-2-[2-(4,5-bis(4-fluorophenyl)-1H-2-imidazolylthio)ethylthio]-1-methylimidazole (3 g) was dissolved in ethanol and treated with aqueous 48% hydrobromic acid. The salt which precipitated upon addition of ether was recrystallized from isopropanol to give the title compound as its dihydrobromide salt, m.p. 202—205°.

$C_{33}H_{24}F_4N_4S_2 \cdot 2\ HBr$

| | | | | |
|---|---|---|---|---|
| Calculated: | 50.91% C; | 3.37% H; | 7.20% N; | 20.53% Br |
| Found: | 50.86% C; | 3.34% H; | 7.22% N; | 20.49% Br |

Example 15

2,2′-[1,3-Propan-2-onediylbis(thio)]bis[4,5-bis(4-fluorophenyl-1H-imidazole]

A solution of 4,5-bis(4-fluorophenyl)-1H-imidazole-2-thione (5.8 g, 0.02 mol) and 1,3-dichloropropanone (1.3 g, 0.01 mol) in ethanol (200 ml) was refluxed overnight. The solvent volume was reduced to one half *in vacuo* and the solution cooled. The resulting precipitate was collected and recrystallized from ethanol to give the title compound as the dihydrochloride salt (hydrate), m.p. 222—223°.

$C_{33}H_{22}F_4N_4OS_2 \cdot 2\ HCl$

| | | | |
|---|---|---|---|
| Calculated: | 56.33% C; | 3.44% H; | 7.96% N |
| Found: | 54.11% C; | 3.27% H; | 7.52% N |

2,2′-[1,3-Propan-2-onediylbis(thio)]bis[4,5-bis(4-fluorophenyl)-1H-imidazole] dihydrochloride is converted to the title compound by the procedure described in Example 1.

Example 16

2,2′-Thiobis[4,5-bis(4-methoxyphenyl)-1H-imidazole]

A mixture of 4,5-bis(4-methoxyphenyl)-1H-imidazole-2-thione (8.0 g, 0.026 mol), 1,2-diiodotetrafluoroethylene (4.5 g, 0.013 mol) and sodium ethoxide (1.74 g, 0.026 mol) in ethanol (100 ml) was refluxed for two hours, then cooled, The precipitate was collected, washed with ethanol and recrystallized from hot acetic acid to give the title compound, m.p. 244—245°.

$C_{34}H_{30}N_4O_4S$

| | | | |
|---|---|---|---|
| Calculated: | 65.57% C; | 4.86% H; | 8.90% N |
| Found: | 65.44% C; | 4.77% H; | 8.66% N |

Example 17

2,2′-[1,2-Propanediylbis(thio)]bis[4,5-bis(4-fluorophenyl)-1H-imidazole]

A solution of 4,5-bis(4-fluorophenyl)-1H-imidazole-2-thione (10 g, 0.035 mol) and 1,2-dibromopropane (3.5 g, 0.017 mol) in N,N-dimethylformamide (25 ml) was refluxed for two hours, then cooled. The precipitate was collected, treated with hot chloroform and recrystallized from isopropanol to give the title compound as its dihydrobromide salt, m.p. 243—245°.

$C_{33}H_{24}F_4N_4S_2 \cdot 2\ HBr$

| | | | |
|---|---|---|---|
| Calculated: | 50.93% C; | 3.37% H; | 7.20% N |
| Found: | 50.69% C; | 3.39% H; | 7.14% N |

2,2'-[1,2-Propanediylbis(thio)]bis[4,5-bis(4-fluorophenyl)-1H-imidazole]dihydrobromide is converted to the title compound by the procedure described in Example 1.

### Example 18

2,2'-[1,3-Propan-2-oldiylbis(thio)]bis[4,5-bis(4-fluorophenyl)-1H-imidazole]

A solution of 4,5-bis(4-fluorophenyl)-1H-imidazole-2-thione (5.8 g, 0.02 mol) and 1,3-dibromo-2-propanol (2.18 g, 0.01 mol) in ethanol (150 ml) was refluxed for 36 hours. The solvent was removed *in vacuo* and the yellow residue was dissolved in chloroform. This solution was passed through silica gel which was washed in turn with chloroform, ether and acetone (400 ml each). The residue, after removing the solvent from the acetone fraction, was recrystallized from acetonitrile to give the title compound as its dihydrobromide salt (hydrate), m.p. 219—221°.

$C_{33}H_{24}F_4N_4OS_2 \cdot 2\ HBr \cdot H_2O$

| | | | |
|---|---|---|---|
| Calculated: | 48.78% C; | 3.47% H; | 6.95% N |
| Found: | 48.67% C; | 3.34% H; | 6.70% N |

2,2'-[1,3-Propan-2-oldiylbis(thio)]bis[4,5-bis(4-fluorophenyl)-1H-imidazole] dihydrobromide is converted to the title compound by the procedure described in Example 1.

### Example 19

2,2'-Thiobis[4,5-bis(4-trifluoromethylphenyl)-1H-imidazole]

A mixture of 4,5-bis(4-trifluoromethylphenyl)-1H-imidazole-2-thione (3.9 g, 0.01 mol), sodium ethoxide (0.68 g, 0.01 mol) and iodine (1.26 g, 0.005 mol) in ethanol (50 ml) was stirred at ambient temperature for two hours. The resulting precipitate was collected and washed with ethanol and then ether to give the title compound m.p. 343—345° (dec.).

$C_{34}H_{18}F_{12}N_4S_2$

| | | | |
|---|---|---|---|
| Calculated: | 52.72% C; | 2.34% H; | 7.23% N |
| Found | 52.61% C; | 2.45% H; | 7.27% N |

### Example 20

2,2'-Thiobis[4,5-bis(4-methylthiophenyl)-1H-imidazole]

A mixture of 4,5-bis(4-methylthiophenyl)-1H-imidazole-2-thione (5.2 g, 0.015 mol), sodium ethoxide (1.0 g, 0.015 mol) and iodine (1.9 g, 0.0075 mol) in ethanol (70 ml) was stirred at ambient temperature for three hours. The resulting solid was collected, washed with ethanol and then ether. Recrystallization from N,N-dimethylformamide give the title compound, m.p. 297—298°.

$C_{34}H_{30}N_4S_6$

| | | | |
|---|---|---|---|
| Calculated: | 59.44% C; | 4.40% H; | 8.15% N |
| Found | 59.32% C; | 4.30% H; | 8.12% N |

### Example 21

| Ingredients | Amounts |
|---|---|
| 2,2'-[1,2-ethanediylbis(thio)]bis[4,5-bis (4-fluorophenyl)-1H-imidazole] | 50 mg. |
| magnesium stearate | 5 mg. |
| lactose | 100 mg. |

The above ingredients are screened, mixed and filled into a hard gelatin capsule.

Example 22

| Ingredients | Amounts |
|---|---|
| 2,2′-[1,2-ethanediylbis(thio)]bis[4,5-bis (4-fluorophenyl)-1H-imidazole] | 100 mg. |
| calcium sulfate dihydrate | 150 mg. |
| sucrose | 20 mg. |
| starch | 10 mg. |
| talc | 5 mg. |
| stearic acid | 3 mg. |

The sucrose, calcium sulfate dihydrate and 2,2′-[1,2-ethanediylbis(thio)]bis[4,5-bis(4-fluorophenyl)-1H-imidazole] are mixed and granulated with 10% gelatin solution. The wet granules are screened, dried, mixed with the starch, talc and stearic acid, screened and compressed into a tablet.

Example 23

| Ingredients | Amounts |
|---|---|
| 2,2′-[1,2-ethanediylbis(thio)][4,5-bis (4-fluorophenyl)-1H-imidazole] | 50 mg. |
| magnesium stearate | 5 mg. |
| lactose | 75 mg. |

The above ingredients are screened, mixed and filled into a hard gelatin capsule.

Similarly, the other compounds of formula I may be formulated into pharmaceutical compositions by the procedures of Examples 21—23.

## Claims

1. A compound of formula (I):—

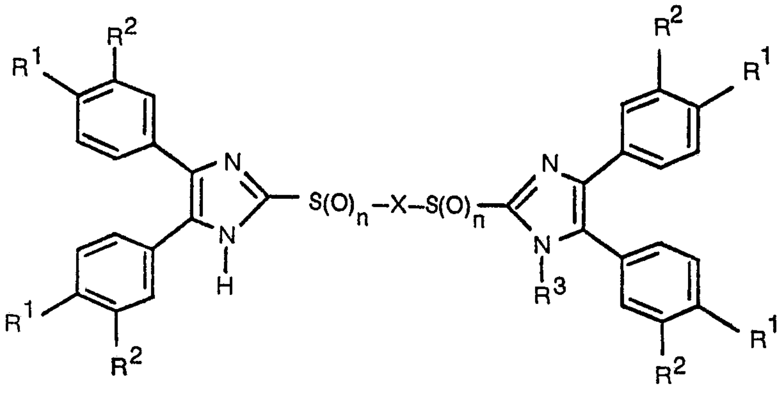

(I)

where $R^1$ is methoxy, methylthio, trifluoromethyl, chloro, fluoro or bromo; $R^2$ is hydrogen or with $R^1$ is methylenedioxy; $R^3$ is hydrogen or methyl; *n* is 0,1 or 2; and X is $-(CH_2)_m$ where *m* is 1 to 4, 1,2-propanediyl, 1,3-propan-2-oldiyl or 1,3-propan-2-onediyl and its pharmaceutically acceptable salts, provided that *n* is 0 when *m* is 1; for use as an anti-arthritic agent.

2. A compound as claimed in claim 1 where $R^1$ is methoxy or fluoro.

3. A compound as claimed in claim 2 where *n* is 0; X is $-(CH_2)_m-$ (*m* being 1 or 2), 1,3-propan-2-oldiyl or 1,3-propan-2-onediyl.

4. 2,2′-[1,2-Ethanediylbis(thio)]bis[4,5-bis-(4-methoxyphenyl)-1H-imidazole] and its pharmaceutically acceptable salts; for use as anti-arthritic agents.

5. A pharmaceutical composition for use as an anti-arthiritic comprising a compound of formula (I) as defined in any one of claims 1 to 4 or a pharmaceutically acceptable salt thereof and a pharmaceutically acceptable carrier.

6. A compound of formula (I):—

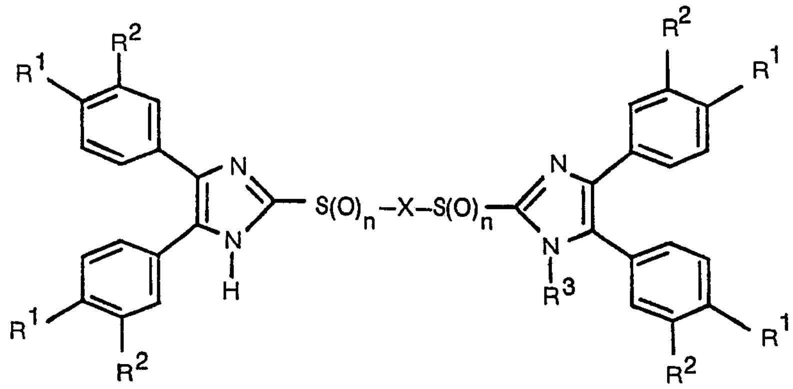

(I)

where $R^1$ is methoxy, methylthio, trifluoromethyl, chloro, fluoro or bromo; $R^2$ is hydrogen or with $R^1$ is methylenedioxy; $R^3$ is hydrogen or methyl; $n$ is 0,1 or 2; and X is $-(CH_2)_m$ where $m$ is 1 to 4, 1,2-propanediyl, 1,3-propan-2-oldiyl or 1,3-propan-2-onediyl and its pharmaceutically acceptable salts, provided that $n$ is 0, when $m$ is 1 and when $R^1$ is methoxy and $n$ is 0, $m$ is not 2.

7. A compound as claimed in claim 6 where $R^1$ is methoxy or fluoro.

8. A compound as claimed in claim 6 where X is $-(CH_2)_m-$ and m is 1 or 2, 1,3-propan-2-oldiyl or 1,3-propan-2-onediyl.

9. 2,2'-[1,2-Ethanediylbis(thio)]bis[4,5-bis-(4-fluorophenyl)-1H-imidazole] and its pharmaceutically acceptable salts.

10. 2,2'-[Methanediylbis(thio)]bis[4,5-bis(4-fluorophenyl)-1H-imidazole] and its pharmaceutically acceptable salts.

11. 2,2'-[1,2-Ethanediylbis(sulfoxy)]bis[4,5-bis(4-fluorophenyl)-1H-imidazole] and its pharmaceutically acceptable salts.

12. 4,5-Bis(4-fluorophenyl)-2-[2-(4,5-bis(4-fluorophenyl)-1H-2-imidazolylthio)ethylthio]-1-methylimidazole and its pharmaceutically acceptable salts.

13. 2,2'-[1,3-Propan-2-oldiylbis(thio)]bis[4,5-bis-(4-fluorophenyl)-1H-imidazole] and its pharmaceutically acceptable salts.

14. 2,2'-[1,3-Propan-2-onediylbis(thio)]bis[4,5-bis-(4-fluorophenyl)-1H-imidazole] and its pharmaceutically acceptable salts.

15. A process for preparing a compound as claimed in any one of claims 6 to 14 which comprises:—

a) reacting a compound of the formula (II):—

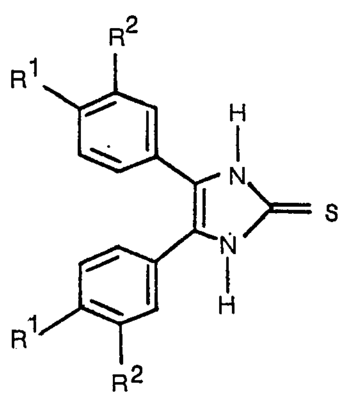

(II)

where $R^1$ and $R^2$ are as defined in claim 6 with a reagent which introduces the group X;

b) optionally reacting the product of a) with a methylhalide and a base; and

c) optionally reacting oxidizing the product of step a) or step b) to a sulphoxide or sulphone.

**Patentansprüche**

1. Verbindungen der Formel (I)

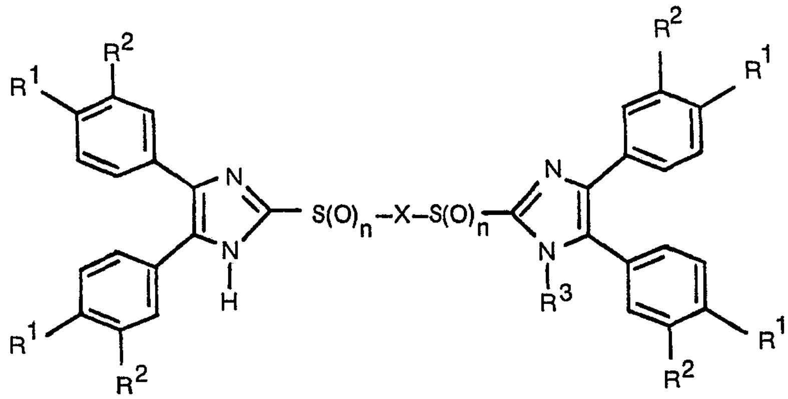

(I)

in der $R^1$ eine Methoxy-, Methylthio- oder Trifluormethylgruppe oder ein Chlor-, Fluor- oder Bromatom bedeutet; $R^2$ ein Wasserstoffatom bedeutet oder zusammen mit $R^1$ eine Methylendioxygruppe darstellt; $R^3$ ein Wasserstoffatom oder eine Methylgruppe bedeutet; *n* 0,1 oder 2 ist und X den Rest $-(CH_2)_m$ bedeutet, in dem *m* 1 bis 4 ist, oder X eine 1,2-Propandiyl-, 1,3-Propan-2-oldiyl- oder eine 1,3-Propan-2-on-diylgruppe darstellt, sowie ihre pharmazeutisch verträglichen Salze, wobei *n* 0 ist, sofern *m* 1 ist; zur Verwendung als antiarthritisch wirksame Mittel.

2. Verbindungen nach Anspruch 1, dadurch gekennzeichnet, daß $R^1$ eine Methoxygruppe oder ein Fluoratom darstellt.

3. Verbindungen nach Anspruch 2, dadurch gekennzeichnet, daß *n* 0 ist, X die Gruppe $-(CH_2)_m-$ bedeutet, wobei *m* 1 oder 2 ist, oder X den 1,3-Propan-2-oldiyl- oder 1,3-Propan-2-ondiylrest darstellt.

4. 2,2′-[1,2-Äthandiylbis-(thio)]-bis-[4,5-bis-(4-methoxyphenyl)-1H-imidazol] und dessen pharmazeutisch verträgliche Salze; zur Verwendung als Antiarthritisch wirksame Mittel.

5. Pharmazeutische Zusammensetzungen zur Verwendung als antiarthritisch wirksame Mittel, enthaltend eine Verbindung der Formel (I) gemäß jedem der Ansprüche 1 bis 4 oder ihrer pharmazeutisch verträglichen Salze und ein pharmazeutisch verträglicher Trägerstoff.

6. Verbindungen der Formel (I):

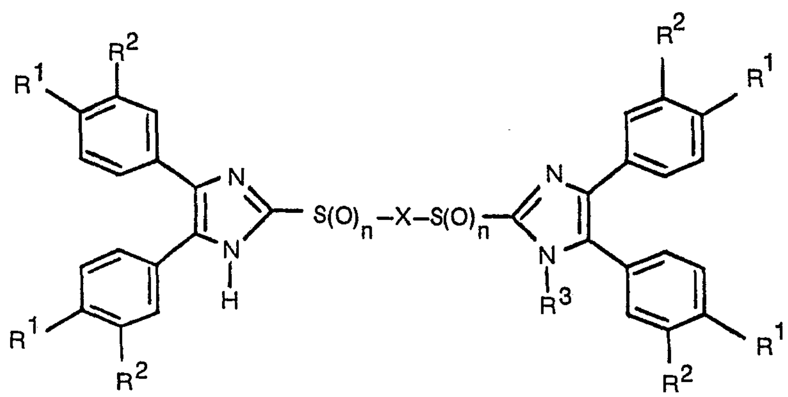

(I)

in der $R^1$ eine Methoxy-, Methylthio- oder Trifluormethylgruppe oder ein Chlor-, Fluor- oder Bromatom bedeutet; $R^2$ ein Wasserstoffatom bedeutet oder zusammen mit $R^1$ eine Methylendioxygruppe darstellt; $R^3$ eine Wasserstoffatom oder eine Methylgruppe bedeutet *n* 0, 1 oder 2 ist und X den Rest $-(CH_2)_m$ bedeutet, in dem *m* 1 bis 4 ist, oder X eine 1,2-Propandiyl-, 1,3-Propan-2-oldiyl- oder eine 1,3-Propan-2-on-diylgruppe darstellt, sowie ihre pharmazeutisch verträglichen Salze, wobei *n* 0 ist, sofern *m* 1 ist und sofern $R^1$ einen Methoxyrest darstellt und *n* 0 ist, *m* nicht den Wert 2 hat.

7. Verbindungen nach Anspruch 6, dadruch gekennzeichnet, daß $R^1$ eine Methoxygruppe oder ein Fluoratom darstellt.

8. Verbindungen nach Anspruch 6, dadurch gekennzeichnet, daß X den Rest $-(CH_2)_m$ darstellt und m 1 oder 2 ist, oder X den 1,3-Propan-2-oldiyl- oder 1,3-Propan-2-ondiylrest bedeutet.

9. 2,2′-[1,2-Äthandiylbis-(thio)]-bis-[4,5-bis-(4-fluorphenyl)-1H-imidazol] sowie dessen pharmazeutisch verträgliche Salze.

10. 2,2'-[Methandiylbis-(thio)]-bis-[4,5-bis-(4-fluorphenyl)-1H-imidazol] sowie dessen pharmazeutisch verträgliche Salze.

11. 2,2'-[1,2-Äthandiylbis-(sulfoxy)]-bis-[4,5-bis-(4-fluorphenyl)-1H-imidazol] sowie dessen pharmazeutisch verträgliche Salze.

12. 4,5-Bis-(4-fluorphenyl)-2-[2-(4,5-bis-(4-fluorphenyl)-1H-2-imidazolylthio)-äthylthio]-1-methylimidazol sowie dessen pharmazeutisch verträgliche Salze.

13. 2,2'-[1,3-Propan-2-oldiylbis-(thio)]-bis-[4,5-bis-(4-fluorphenyl)-1H-imidazol] sowie dessen pharmazeutisch verträgliche Salze.

14. 2,2'-[1,3-Propan-2-ondiylbis-(thio)]-bis-[4,5-bis-(4-fluorphenyl)-1H-imidazol] sowie dessen pharmazeutisch verträgliche Salze.

15. Verfahren zur Herstellung von Verbindungen nach jedem der Ansprüche 6 bis 14, dadurch gekennzeichnet, daß man

a) eine Verbindung der Formel (II)

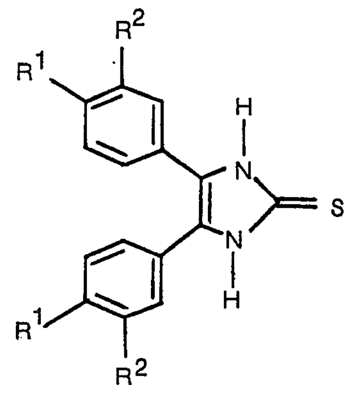

(II)

in der $R^1$ und $R^2$ die in Anspruch 6 angegebene Bedeutung haben, mit einem Umsetzungspartner zur Umsetzung bringt, der die Gruppe X einführt;

b) das gemäß a) erhaltene Produkt gegebenenfalls mit einem Methylhalogenid und einer Base umsetzt; und

(c) das gemäß Stufe a) oder Stufe b) erhaltene Produkt gegebenenfalls zu einem Sulfoxid oder einem Sulfon oxidiert.

**Revendications**

1. Composé de formule (I)

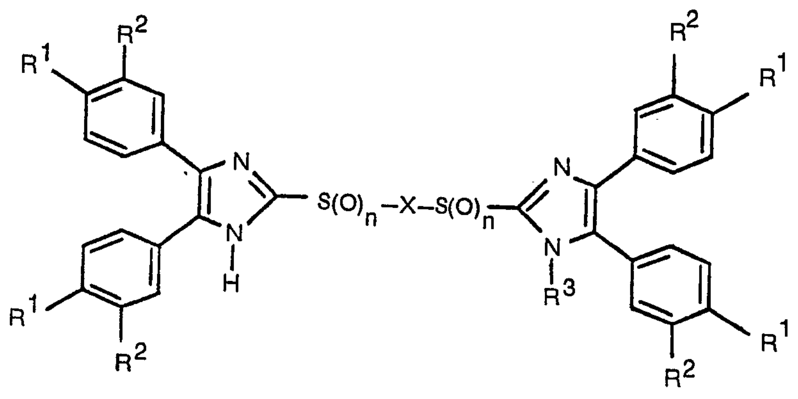

(I)

dans laquelle $R^1$ est méthoxy, méthylthio, trifluorométhyle, chloro, fluoro ou bromo; $R^2$ est l'hydrogène ou, avec $R^1$, forme un groupe méthylènedioxy; $R^3$ est l'hydrogène ou un groupe méthyle; n est égal à 0, 1 ou 2; et X est $-(CH_2)_m-$ où m est égal à 1 à 4, 1,2-propanediyle, 1,3-propan-2-oldiyle ou 1,3-propan-2-onediyle et ses sels pharmaceutiquement acceptables, à condition que n soit égal à 0 lorsque m est égal à 1; pour utilisation comme agent anti-arthritique.

2. Composé selon la revendication 1, dans lequel $R^1$ est méthoxy ou fluoro.

3. Composé selon la revendication 2, dans lequel n est égal à 0; X est $-(CH_2)_m-$ (m étant 1 ou 2), 1,3-propan-2-oldiyle ou 1,3-propan-2-onediyle.

4. 2,2'-[1,2-éthanediylbis(thio)]-bis-[4,5-bis-(4-méthoxyphényl)-1H-imidazole] et ses sels pharmaceutiquement acceptables; pour utilisation comme agents arthritiques.

5. Composition pharmaceutique pour utilisation comme agent anti-arthritique comprenant un composé de formule (I) comme défini dans l'une quelconque des revendications 1 à 4 ou un sel pharmaceutiquement acceptable de ce composé et un véhicule pharmaceutiquement acceptable.

6. Composé de formule (I)

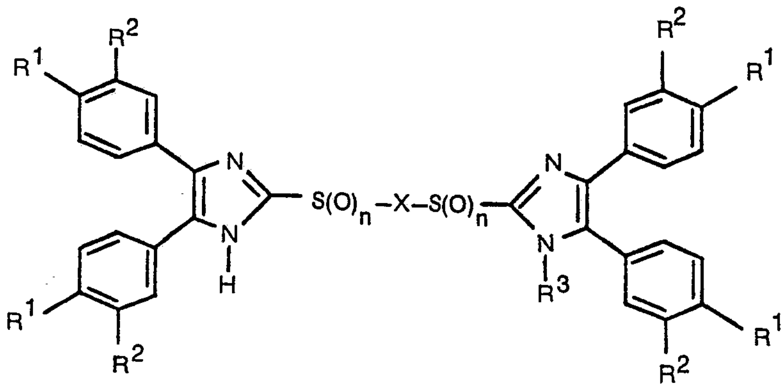

(I)

dans laquelle $R^1$ est un groupe méthoxy, méthylthio, trifluorométhyle, chloro, fluoro ou bromo; $R^2$ est l'hydrogène ou, avec $R^1$ forme un groupe méthylènedioxy; $R^3$ est l'hydrogène ou un groupe méthyle; n est égal à 0, 1 ou 2; et X est $-(CH_2)_m-$ où m est égal à 1 à 4, 1,2-propanediyle, 1,3-propan-2-oldiyle ou 1,3-propan-2-onediyle, et ses sels pharmaceutiquement acceptables, à condition que n soit égal à 0 lorsque m est égal à 1 et que, lorsque $R^1$ est le groupe méthoxy et n est égal à 0, m soit différent de 2.

7. Composé selon la revendication 6, dans lequel $R^1$ est méthoxy ou fluoro.

8. Composé selon la revendication 6, dans lequel X est $-(CH_2)_m-$ et m est égal à 1 ou 2, 1,3-propan-2-oldiyle ou 1,3-propan-2-onediyle.

9. 2,2'-[1,2-éthanediylbis(thio)]-bis-[4,5-bis-(4-fluorophényl)-1H-imidazole] et ses sels pharmaceutiquement acceptables.

10. 2,2'-[méthanediylbis(thio)]-bis-[4,5-bis-(4-fluorophényl)-1H-imidazole] et ses sels pharmaceutiquement acceptables.

11. 2,2'-[1,2-éthanediylbis(sulfoxy)]-bis-[4,5-bis-(4-fluorophényl)-1H-imidazole] et ses sels pharmaceutiquement acceptables.

12. 4,5-bis-(4-fluorophényl)-2-[2-(4,5-bis-(4-fluorophényl)-1H-2-imidazolylthio)éthylthio]-1-méthylimidazole et ses pharmaceutiquement acceptables.

13. 2,2'-[1,3-propan-2-oldiylbis(thio)]-bis-[4,5-bis-(4-fluorophényl)-1H-imidazole] et ses sels pharmaceutiquement acceptables.

14. 2,2'-[1,3-propan-2-onediylbis(thio)]-bis-[4,5-bis-(4-fluorophényl)-1H-imidazole] et ses sels pharmaceutiquement acceptables.

15. Procédé de préparation d'un composé selon l'une quelconque des revendications 6 à 14, qui consiste:

(a) à faire réagir un composé de formule (II):

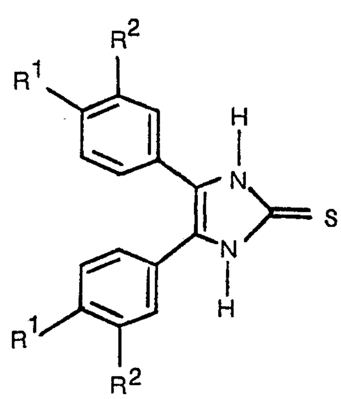

(II)

dans laquelle $R^1$ et $R^2$ sont tels que définis dans la revendication 6, avec un réactif qui introduit le groupe X;

(b) à faire réagir éventuellement le produit de a) avec un halogénure de méthyle et une base; et

(c) à oxyder éventuellement le produit du stade a) ou du stade b) en un sulfoxyde ou une sulfone.